# EUROPEAN PATENT SPECIFICATION

(11) **EP 1 969 999 B1**
(45) Date of publication and mention of the grant of the patent: **10.07.2013**
(21) Application number: 08101033.2
(22) Date of filing: 29.01.2008
(51) Int. Cl.: A61B 5/00

(54) **Miniature, wireless apparatus for processing physiological signals and use thereof**
Miniaturfunkvorrichtung zur Verarbeitung physiologischer Signale und Verwendung davon
Appareil miniature sans fil pour traiter les signales physiologiques et utilisation associée.

(30) Priority: 16.03.2007 TW 96109119
(43) Date of publication of application: 17.09.2008
(73) Proprietor: National Yang-Ming University, Taipei City 112 (TW)
(72) Inventor: Kuo, Terry B.J., Ji-an Township, Hualien County (TW); Yang, Cheryl C.H., Ji-an Township, Hualien County (TW)
(74) Representative: Lang, Christian

(56) References cited:
- WO-A-97/18639
- US-A1- 2001 034 475
- US-B1- 6 188 715

## Description

### FIELD OF THE INVENTION

The present invention relates to a miniature, wireless apparatus for processing physiological signals and use thereof.

### DESCRIPTION OF PRIOR ART

Physiological signals such as ECG, EEG, breath, and body temperature are signs of health. When combining and analyzing the above physiological signals as well as the electromyogram, one can obtain the indications of sleep and autonomic nervous system. Collection and analysis of these physiological signals facilitates the understanding and medical application of numerous medical information. Particularly, the design of wireless remote measurement can reflect the physiological phenomena with high accuracy and low interference and provide important information for precise understanding of various physiological functions.

Sleep medicine has underwent a breakthrough in the past five years. Some chronic sleep-related diseases including obstructive sleep apnea are getting more and more attention. Many medical researches also indicate that sleep troubles are probably one of the factors of hypertension. The number of sleep-related research and clinical examination has remarkably increased in recent years. It is thus evident that the sleep-related research is one of the emphases in future medical development. However, the slow progress of sleep research at present medical environment made the sleep a key leak in the clinical healthcare. So far, the deficiency and poor establishment of the long-term monitoring instruments and analyzing tools resulted in the low willingness of patients as well as the limitation of the sleep-related medical development.

The key defects of existing sleep-medicine detecting system are described as follows:
(1) the constraint of traditional wired system:
   The existing long-term physiological detecting system was mainly established on the basis of wired transmission technologies. Patients should paste a lot of electrodes on his or her body parts, and then the electrodes are connected to the signal amplifier via conducting wire for digital-to-analog conversion and digital signal processing. It was very awkward to operate the traditional wired system. The movement of patients was highly constrained due to the wiring all over the body. Even going to the toilet was inconvenient under the examination. As a result of all above disamenities, many patients hesitate to go for examination or refuse to cooperate with doctors for long-term inspection.
(2) the high price and difficulty of operation of traditional instruments :
   Due to the need of professional technicians for operating long-term physiological signal detection system, the efficiency of sleep examination in hospitals is low. After a period of training the sleep examination could be administered smoothly.
(3) Recently, a few wireless instruments are under development, but the convenience of operation need to be improved.

Some manufacturers have launched so-called wireless system on the market, but most of the wireless systems are still limited by the tiresome conducting wires. The electrodes should be connected to the host by wire. After amplification and digital-to-analog conversion, the digital signals are then emitted by microcontroller and radio module. The whole system must be connected by conducting wires. Although the above system contributes to some improvement and convenience for the patients, those wires to some extent still restrict the movement of patients. Moreover, the wires themselves are the origin of various noise signals, causing the reduced accuracy of the examination result. In addition, the existing systems are unable to receive and process different physiological signals in one channel simultaneously, resulting in the squander of bandwidth and electricity. The fact that bulky instruments are not easy to carry is still another problem.

To sum up, the existing sleep detection systems have some inherent disadvantages. The conducting wires not only lead to the inconvenience of patients but inevitably increase the origins of noise signal. Additionally, these instruments are expensive, difficult to operate, and too bulky to monitor the patients' condition momentarily. Hence, developing a totally wireless, inexpensive, and handy physiological signal recording and examination system for personal operation and long-term monitoring is necessary and urgent. Then a physiological signal examination system with more convenience and less noise signals can be realized for monitoring patients' condition, evaluating the effect of operation, and further understanding the course of disease. Patent No.090128786 of R.O.C provided a sensor for sitnultaneously detecting electrocardiogram signal, pulsation, and acoustic wave from the neck. Patent application No. 091106492 of R.O.C provided a non-invasive autonomic nervous monitoring system and use thereof. Patent application No.092117250 of R.O.C. provided an electrocardiogram signal converter and analog-to-digital converting device thereof. US patent No. 6,360,117 provided an electrocardiogram signal collecting apparatus.

Further signal collecting apparatuses are known from US 2001/034475 A1 and WO 97/18639 A. US 2001/034475 A1 discloses a medical telemetry system for collecting real-time physiologic data of patients. WO 97/18639 discloses a two-way medical telemetry system for displaying and monitoring physiologic and other patient data at a central location.

### SUMMARY OF THE INVENTION

The present invention provides a miniature, wireless apparatus for processing physiological signals comprising a signal-receiving element and a signal-processing element, wherein said signal-receiving element receives plural of signals input from external environment and transmits the signals to said signal-processing element; and said signal-processing element divides the receiving-time into n equal intervals and corresponds each divided time-interval to a signal detected by one sensor.

The present invention further provides a method for processing physiological signals comprising receiving the signals by signal-receiving element, dividing the receiving-time into n equal intervals by signal-processing element and corresponding each divided time-interval to a signal which is detected by one sensor.

### BRIEF DESCRIPTION OF THE DRAWINGS

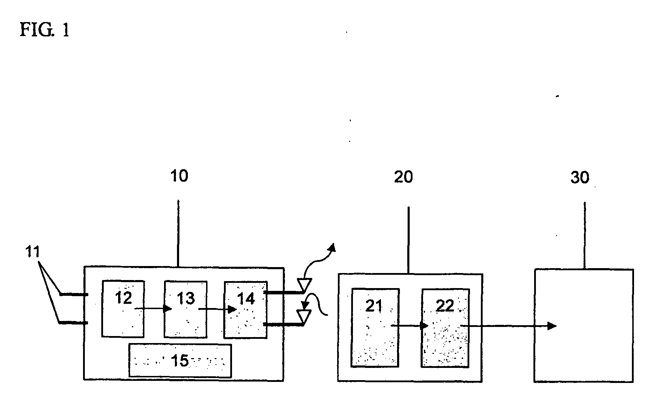

Fig.1 indicates the apparatus for processing physiological signals combined with the signal sensor and the signal recorder.

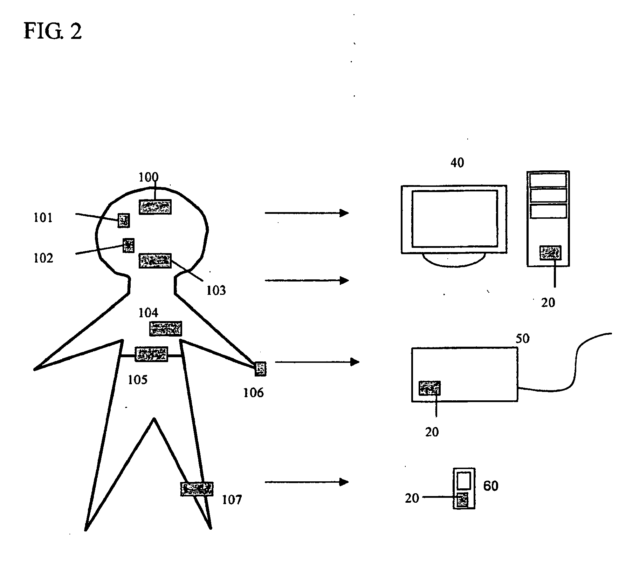

Fig.2 indicates that multiple signal sensors transmits the detected signals synchronously to the apparatus for processing physiological signals.

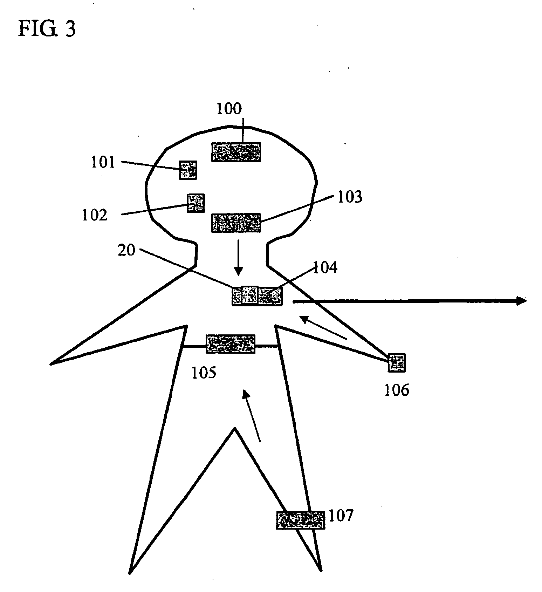

Fig.3 indicates that the apparatus for processing physiological signals is integrated on a signal sensor.
Fig.4 indicates that the signal-processing element divides the receiving-time into 8 equal intervals and corresponds each divided time-interval to different signals detected.

### Description of major parts in the present invention

10: signal sensor
11: electrode
12: amplifier
13: microcontroller
14: transceiver module
15: power supply
20: signal processing device
21: signal-receiving element
22: signal-processing element
30 : signal recorder
100 : wireless electroencephalogram sensor
101 : wireless electrooculogram sensor
102 : wireless electromyogram sensor
103 : wireless temperature sensor
104 : wireless electrocardiogram sensor
105 : miniature tension sensor
106 : blood oxygen saturation sensor
107 : miniature acceleration sensor
40 : personal computer
50 : Internet
60 : GSM mobile phone

### DETAILED DESCRIPTION OF THE INVENTION

### Term Definition

### Electroencephalogram (EEG)

A chart of the brain waves picked up by the electrodes placed on the scalp. Changes in brain wave activity can be an indication of REM sleep, consciousness, and nervous system disorders.

### Electrooculogram (EOG)

EOG is a technique for measuring the resting potential of the retina. The resulting signal is called the electrooculogram. The main applications are in ophthalmological diagnosis and in recording eye movements. Unlike the electroretinogram, the EOG does not represent the response to individual visual stimuli.

### Electromyogram (EMG)

An electromyogram (EMG) is a test that is used to record the electrical activity of muscles. When muscles are active, they produce an electrical current. This current is usually proportional to the level of the muscle activity. An EMG is also referred to as a myogram.

### Electrocardiogram (ECG or EKG)

The electrocardiogram (ECG or EKG) is a noninvasive test that is used to reflect underlying heart conditions by measuring the electrical activity of the heart. By positioning leads (electrical sensing devices) on the body in standardized locations, information about many heart conditions can be learned by looking for characteristic patterns on the ECG.

### Firmware

In computing, firmware is software that is embedded in a hardware device. It is often provided on flash memory or as a binary image file that can be uploaded onto existing hardware by a user.

The present invention provides an apparatus for processing physiological signal comprising a signal-receiving element and a signal-processing element. The signal-receiving element receives plural of signals input from external sensors and transmits the signals to the signal-processing element. The signal-processing element then divides the receiving-time into n equal intervals and corresponds each divided time-interval to a signal detected by one sensor.

In the present invention, n is ranged from 1 to 50. In the preferred embodiment, n is ranged from 1 to 30. In the more preferred embodiment, n is ranged from 1 to 20. In the best embodiment, n is ranged from 1 to 10.

The present invention further comprises one or more signal sensors which consist of electrode pair, amplifier, microcontroller, transceiver module, and power supply.

The electrode pair of the signal sensor is differential and comprises a positive electrode and a negative electrode which are connected to a person under test to collect a pair of physiological signals.

The amplifier of the signal sensor comprises a pair of input filters, a differential amplifier, and an output filter. The physiological signals collected from the positive electrode and the negative electrode have noise filtered out by the input filters to increase the signal-to-noise ratio, and then the physiological signals are differentially amplified by the differential amplifier. The differential amplifier attenuates the common mode noise of the pair of physiological signals, and simultaneously amplifies the differential part of the pair of physiological signals with appropriate magnification, so as to match the voltage range of the analog-to-digital conversion of the microcontroller.

The output filter filters out an amplified physiological signal that is over the Nyquist frequency (i.e., twice the sampling frequency of the analog-to-digital conversion of the microcontroller). Moreover, the impedance of the input end of the amplifier is larger than 200 kΩ, so as to prevent the leakage current caused by an operational error. The input filter and the output filter can be implemented by passive elements such as resistors or capacitors. The differential amplifier can be implemented by an operational amplifier or an instrumentation amplifier of the integrated circuit.

The microcontroller of the signal sensor comprises an analog-to-digital conversion unit and a digital signal processing unit. The analog-to-digital conversion unit performs an analog-to-digital conversion for the amplified physiological signal generated from the amplifier with the appropriate voltage resolution and sampling frequency, and then the digital signal processing unit performs a data compression for a digital physiological signal generated by the analog-to-digital conversion unit.

The transceiver module comprises a wireless transceiver and a modulator/demodulator. The input end of the transceiver module, being connected to the microcontroller, is a serial or parallel digital channel for receiving a digital physiological signal generated from the microcontroller. Then the modulator modulates the digital physiological signal compressed by the digital signal processing unit to a modulated physiological signal with the carrier frequency of 2.4 GHz. The modulated physiological signal is sent to a far end by the wireless transceiver in the form of a wireless physiological signal. Meanwhile, the wireless transceiver also receives a wireless signal from the far end, and then the wireless signal is demodulated by the demodulator to a digital data signal, and the digital data signal is transmitted to the microcontroller through the digital channel. The wireless signal sent from the far end comprises a control signal of the signal sensors and an acknowledgement signal sent by a signal-receiving element of the far end. The transceiver module performs wireless transmission and reception using the international industry, science, and medical (ISM) exclusive frequency band.

The signal receiving element of the apparatus provided in the present invention will guide the signal to the corresponding time-interval if the transceiver module doesn't send signal at corresponding time-interval. This guiding function makes it possible to corresponding detected signals from different sensors to the corresponding time-interval accurately. Therefore, a highly precise signal recording can be realized.

In the present invention, the radio interface is used as the transceiver module of signal sensor. The radio interface not only converts the digital physiological signals into radio signals as well as sending them but receives the radio signals inputted from outside.

In the present invention, the physiological signals include physiological signals transmitted by wired or wireless tools. Moreover, the physiological signals are human physiological signals including electrocardio-signal, electroencephalo-signal, electromyo-signal, electrooculo-signal, body temperature, tensile signal, and accelerative signal.

The signal sensor provided by the present invention is selected from the group consisting of wired/wireless electrocardiogram sensor, wired/wireless electroencephalogram sensor, wired/wireless temperature sensor, wired/wireless electromyogram sensor, miniature wired/wireless tension sensor, and wired/wireless acceleration sensor.

There are many open circuits of above sensors for free use and reference, including the collection of electro-signals, body temperature, blood oxygen concentration, and the circumference of thorax.

The apparatus provided by present invention further comprises a remote signal recorder. The remote signal recorder is hard disc, floppy disc, miniature hard disc, or flash memory card which records and saves a large number of various processed physiological signals via wireless transmission for further analysis.

The apparatus provided by the present invention is carried out by micro-computer system such as personal computer, notebook computer, radio station, or personal digital assistant, which can further analyze the collected data or deliver the collected data to other signal-receiving elements. Additionally, the results of analysis can be transmitted to other micro-computer systems via the internet. The apparatus provided by the present invention also collects, saves, and transmits the sleeping data as a peripheral device of a portable computer system. Data analysis in the present invention is carried out by sleep-analyzing algorithm and autonomic nervous-analyzing algorithm.

The signal-receiving element of the apparatus can further send a feedback signal to transceiver module in all of signal sensors, making the transceiver module send signals in a synchronized pattern. The synchronization favors the subsequent comparison and calculation in signal analysis and therefore a more accurate evaluation and diagnosis can be obtained.

In the present invention, the physiological signal processing apparatus can be further integrated into the signal sensors. By the concept of System-on-a-Chip (SoC), various functions including signal detection, reception, and processing can be totally integrated on a single chip set. Consequently, the trend of miniaturization and multi-function in electronic instruments can be realized. The radio signals emitted by other signal sensors can be simultaneously collected, combined, and saved in the remote signal recorder by the integrated system. An entirely wearable physiological signal monitoring system can be obtained. All the wireless physiological signal sensors and the signal recorder are put on the patient so that one can go around at will. Moreover, all kinds of physiological signals such as EEG and ECG under sleeping can be analyzed with minimum disturbance to the activities of daily livings. The system can be used to monitor the course of chronic diseases and to evaluate the effects of operation, the sleeping quality, and the autonomic nervous function.

The present invention further provides a method for processing physiological signals comprising a step of receiving the signals and a step of dividing the receiving-time into n equal intervals by signal-processing element and corresponding each divided time-interval to a signal which is detected by one sensor.

In the present invention, n is ranged from 1 to 50. In the preferred embodiment, n is ranged from 1 to 30. In the more preferred embodiment, n is ranged from 1 to 20. In the best embodiment, n is ranged from 1 to 10.

The method provided by the present invention can be used to synchronously collect the signals emitted by several signal sensors in a single frequency channel. In addition to the efficient use of limited bandwidth, the method combined with the existent digital wireless transmission technologies would reaches the optimized application. Due to the simplified structures, the synchronous data-collection can be carried out in a more power-saving condition.

The method provided by the present invention further comprises a signal-detecting step by signal sensors and a signal-recording step by a signal recorder. The signal-receiving element can further send a feedback signal to the transceiver module in all of the signal sensors, making transceiver module send signals in a synchronized pattern.

The method provided by the present invention can be combined with wireless physiological detection technology, synchronous emission/receiving technology, synchronous recording/saving technology, and sleep-analyzing algorithm in order to fulfill a totally wireless and simple-to-use physiological signal monitoring system for accurate and real time analysis. The monitoring system can be used in evaluation of sleep quality, diagnosis of sleep obstacles, assessment of effect of hypnotics, evaluation of side effect to sleep and autonomic nervous function caused by various drugs, assessment of influences on sleep and autonomic nervous function due to various regimen and health-improving methods, evaluation of influences on sleep and autonomic nervous function caused by taking health food, and assessment of sleeping condition of elders and new-born infants.

### Examples

The following examples are offered by way of illustration and not by way of limitation.

### Example 1: Synchronous signal collection by multiple wireless sensors

Various signal sensors 10 were pasted on the particular body parts for collecting different physiological signals. The wireless electroencephalogram sensor 100 was put on forehead for collecting EEG. The wireless electrooculogram sensor 101 was put on the canthus for collecting EOG The wireless electromyogram sensor 102 was put on the corners of the mouth or chin for collecting EMG. The wireless temperature sensor 103 was put beneath the nostrils for collecting the temperature of breath as a indication of snort. The wireless electrocardiogram sensor 104 was put on the chest for collecting ECG. The miniature tension sensor 105 was put on the pectoral for evaluating the respiratory function. The blood oxygen saturation sensor 106 was put on the finger for detecting the blood oxygen saturation. The miniature acceleration sensor 107 was put on the body or legs for collecting accelerative signals as a quantitative indication of movement or postures.

### Example 2: Signal receiving of multiple wireless sensors

In order to obtain a high-quality result of physiological signal analysis, the signals of multiple signal sensors 10 should be emitted and received in a synchronous manner. In the present invention, the firmware was embedded on the microcontroller 13 of every signal sensor 10. Not only the transceiver module 14 was involved in the present invention but the signal receiving element 21 received all the emitting signals and carried out the synchronous control. Furthermore, flash memory was built in the signal sensor 10 of the present invention for extended storage of the collected physiological signal and increased flexibility.

### Example 3: Signal recording of multiple wireless sensors

The signals collected by the signal sensors 10 were transmitted to signal-receiving element 21 by wireless transmission, or recorded in the remote signal recorder 30 of signal sensors 10. In spite of the convenience brought by the wireless transmission, the wireless signal transmission was prohibited in many places such as hospitals. Interferences in the environment may also reduce the quality of wireless signal transmission. The signal recorder 30 of the present invention contained a Non-Volatile Random Access Memory for recording various physiological signals. Even if the power failure or poor signal transmission occur, the signals recorded by the signal recorder 30 will not be affected. The flexibility of use and the possibility of long-term signal collection of were further increased by the continuity of the recording function provided by the physiological signal monitoring system.

### Example 4: The principle of signal processing of multiple wireless sensors

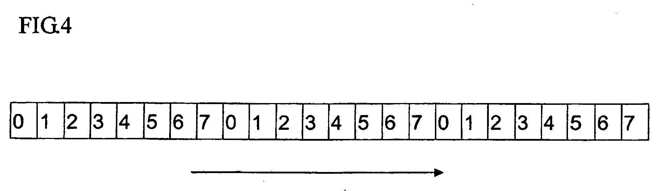

The signal-processing element 22 in the signal processing device 20 divided the receiving-time into n equal intervals and assigned numbers to the intervals. In the best embodiment, there are eight different signal sensors 10 so the signal-processing element 22 divided the receiving-time into eight equal time intervals and each of the intervals was assigned from 0 to 7(Fig.4). Each time interval could only receive signals emitted from one particular signal sensor 10. For instance, time interval 0 could only receive the signals emitted from the signal sensor 100; time interval 1 could only receive the signals emitted from the signal sensor 101; time interval 2 could only receive the signals emitted from the signal sensor 102, and so forth.

If any one of the signal sensors 10 failed to send signals at corresponding time-interval, the signal receiving element 21 will guide the signal to the corresponding time-interval. In addition, the signal-receiving element 21 could further send a feedback signal to the transceiver module 14 in all of the signal sensors 10, making the transceiver module 14 send signals in a synchronized pattern. The whole course of synchronization was conducted by the signal receiving element 21, and all of the signal sensors 10 were also finely tuned by the signal receiving element 21. Accordingly, the signals could be still kept in a perfectly synchronized pattern even after a long-term rewording.

## Claims

1. An apparatus for processing physiological signals comprising:
several wireless sensors (10), each comprising an electrode pair (11), an amplifier (12), a microcontroller (13), a transceiver module (14) and power supply (15), in which the electrode pair (11) is differential and comprises a positive electrode and a negative electrode, connected to a person under test to collect a pair of physiological signals;
a signal processing device (20), comprising:
a signal-receiving element (21), and
a signal-processing element (22); and
a signal recorder (30) comprising a non-volatile random access memory, wherein the signal-receiving element (21) is configured to receive during a receiving-time a plurality of the pair of physiological signals emitted by the several signal sensors (10) in a single frequency channel and wherein the signal-receiving element (21) is configured to transmit the plurality of the pair of physiological signals to the signal-processing element (22);
wherein the signal-processing element (22) is configured to divide the receiving-time in the signal frequency channel into n equal intervals and to correspond each divided time-interval to each of the several sensors (10);
wherein the signal receiving element (21) is configured to correspond one of said pairs of controlled physiological signals to the time-interval corresponding to the respective sensor (10), , if the transceiver module (14) of said respective sensor (10) does not send the collected pair of physiological signals at the corresponding time-interval;
wherein the signal-receiving element (21) is further configured to further send a feedback signal to the transceiver module (14) of each of the several wireless sensors (10), making each of the transceiver module (14) send the pair of physiological signals in a synchronized manner; and
wherein the apparatus is configured to record the processed physiological signals to the signal recorder (30).

2. The apparatus as claimed in claim 1, wherein n is ranged from 1 to 50.

3. The apparatus as claimed in claim 1, wherein the signal sensor (10) is selected from the group consisting of a wireless electrocardiogram sensor (104), a wireless electroencephalogram sensor (100), a wireless temperature sensor (103), a wireless electromyogram sensor (102), a miniature tension sensor (105), and an acceleration sensor.

4. The apparatus as claimed in claim 1, wherein the transceiver module (14) is a radio interface.

5. The apparatus as claimed in claim 1, wherein said signal recorder (30) is a hard disc, a floppy disc, a miniature hard disc, or a flash memory card.

6. The apparatus as claimed in claim 1, which is carried out by a microcomputer system such as a personal computer (40), a notebook computer, a radio station, or a personal digital assistant (60).

7. The apparatus as claimed in claim 1, which can further analyze the collected data or deliver the collected data to other signal-receiving elements.

8. The apparatus as claimed in claim 7, wherein the data analysis is carried out by a sleep-analyzing algorithm and an autonomic nervous-analyzing algorithm.

9. The apparatus as claimed in claim 1, wherein the signal processing device (20) and the several wireless sensors (10) are integrated by a system-on-a-chip.

10. The apparatus as claimed in claim 1, wherein the pair of physiological signals is a human physiological signal.

11. A method for processing physiological signals comprising:
providing several wireless sensors (10), each comprising an electrode pair (11), an amplifies (12), a microcontroller (13), a transceiver module (14) and power supply (15), in which the electrode pair (11) is differential and comprises a positive electrode and a negative electrode, connected to a person under test to collect a pair of physiological signals;
providing a signal processing device (20) comprising:
a signal-receiving element (21), and
a signal-processing element (22); and
providing a signal recorder (30) comprising a non-volatile random access memory;
wherein the signal-receiving element (21) receives during a receiving time a plurality of the pair of physiological signals emitted by the several wireless signal sensors (10) in a single frequency channel and transmits the plurality of the pair of physiological signals to the signal-processing element (22);
wherein the signal-processing element (22) divides the receiving-time in the single frequency channel into n equal intervals and corresponds each divided time-interval to each of the wireless sensors (10);
wherein the signal receiving element (21) correspond one of said pairs of controlled physiological signals to the time-interval corresponding to the respective sensor (10), , if the transceiver module (14) of said respective sensor sensor (10) does not send the collected pair of physiological signals at the corresponding time-interval, wherein the signal-receiving element (21) further sends a feedback signal to the transceiver module (14) of each of the several wireless sensors (10), making each transceiver module (14) send the pair of physiological signals in a synchronized manner; and
wherein the processed physiological signals are recorded to the signal recorder (30).

12. The method as claimed in claim 11, which further comprises a signal-detecting step by each of the several wireless signal sensors (10).

13. The method as claimed in claim 11, which can be used in evaluation of sleep quality, diagnosis of sleep obstacles, assessment of effect of hypnotics, evaluation of side effect to sleep and autonomic nervous function caused by various drugs, assessment of influences on sleep and autonomic nervous function due to various regimen and health-improving methods, evaluation of influences on sleep and autonomic nervous function caused by taking health food, and assessment of sleeping condition of elders and new-born infants.

## Patentansprüche

1. Apparat zum Verarbeiten von physiologischen Signalen, der umfasst:
mehrere drahtlose Sensoren (10), von denen jeder ein Elektrodenpaar (11), einen Verstärker (12), eine Mikrosteuerung (13), ein Übertragungsmodul (17) und eine Energieversorgung (15) umfasst, bei welchen das Elektrodenpaar (11) unterschiedlich ist und eine positive Elektrode und eine negative Elektrode umfasst, die mit einer Testperson verbunden werden, um ein Paar von physiologischen Signalen aufzunehmen;
eine Signalverarbeitungsvorrichtung (20), die umfasst:
ein Signalempfangselement (21), und
ein Signalverarbeitungselement (22); und
einen Signalaufzeichner (30), der einen nichtflüchtigen Schreib-Lese-Speicher umfasst,
wobei das Signalempfangselement (21) dafür ausgelegt ist, während einer Aufnahmezeit eine Vielzahl Paare von physiologischen Signalen zu empfangen, die durch die mehreren Signalsensoren (10) in einem Einzelfrequenzkanal ausgegeben werden, und wobei das Signalempfangselement (21) darauf ausgelegt ist, die Vielzahl der Paare von physiologischen Signalen an das Signalverarbeitungselement (22) zu übertragen;
wobei das Signalverarbeitungselement (22) darauf ausgelegt ist, die Empfangszeit in dem Signalfrequenzkanal in n gleiche Intervalle zu teilen und jedes der getrennten Zeitintervalle jedem der mehreren Sensoren (10) zuzuordnen;
wobei das Signalempfangselement (21) darauf ausgelegt ist, eines der Paare von aufgenommenen physiologischen Signalen einem Zeitintervall zuzuordnen, das dem jeweiligen Sensor (10) entspricht, wenn das Übertragungsmodul (14) des jeweiligen Sensors (10) nicht das aufgenommene Paar von physiologischen Signalen zu dem entsprechenden Zeitintervall sendet;
wobei das Signalempfangselement (21) ferner darauf ausgelegt ist, des Weiteren ein Rückkopplungssignal an das Übertragungsmodul (14) von jedem der mehreren drahtlosen Sensoren (10) zu senden, um zu bewirken, dass jedes der Übertragungsmodule (14) das Paar von physiologischen Signalen in einer synchronisierten Weise sendet; und
wobei der Apparat darauf ausgelegt ist, die verarbeiteten physiologischen Signale in dem Signalaufzeichner (30) zu speichern.

2. Apparat nach Anspruch 1, wobei n im Bereich von 1 bis 50 ist.

3. Apparat nach Anspruch 1, wobei der Signalsensor (10) aus der Gruppe ausgewählt ist, die einen drahtlosen Elektrokardiogrammsensor (104), einen drahtlosen Elektroenzephalogrammsensor (100), einen drahtlosen Temperatursensor (103), einen drahtlosen Elektromyogrammsensor (102), einen Miniaturspannungssensor (105) und einen Beschleunigungssensor umfasst.

4. Apparat nach Anspruch 1, wobei das Übertragungsmodul (14) eine Funkschnittstelle ist.

5. Apparat nach Anspruch 1, wobei der Signalaufzeichner (30) eine Festplatte, eine Diskette, eine Miniaturfestplatte oder eine Flashspeicherkarte ist.

6. Apparat nach Anspruch 1, der durch ein Computersystem wie beispielsweise einen Personalcomputer (40), ein Notebook, eine Funkstation oder einen elektronischen Organizer (60) ausgeführt ist.

7. Apparat nach Anspruch 1, der ferner die aufgenommenen Daten analysieren kann oder die aufgenommenen Daten an andere Signalempfangselemente übertragen kann.

8. Apparat nach Anspruch 7, wobei die Datenanalyse durch einen Schlafanalysealgorithmus und einen autonomen Nervenanalysealgorithmus ausgeführt wird.

9. Apparat nach Anspruch 1, wobei die Signalverarbeitungsvorrichtung (20) und die mehreren drahtlosen Sensoren (10) in einem Ein-Chip-System integriert sind.

10. Apparat nach Anspruch 1, wobei das Paar von physiologischen Signalen ein menschliches physiologisches Signal ist.

11. Verfahren zum Verarbeiten physiologischer Signale, das umfasst:
Bereitstellen mehrerer drahtloser Sensoren (10), von denen jeder ein Elektrodenpaar (11), einen Verstärker (12), eine Mikrosteuerung (13), ein Übertragungsmodul (17) und eine Energieversorgung (15) umfasst, bei welchen das Elektrodenpaar (11) unterschiedlich ist und eine positive Elektrode und eine negative Elektrode umfasst, die mit einer Testperson verbunden werden, um ein Paar von physiologischen Signalen aufzunehmen;
Bereitstellen eine Signalverarbeitungsvorrichtung (20), die umfasst:
ein Signalempfangselement (21), und
ein Signalverarbeitungselement (22); und
Bereitstellen eines Signalaufzeichners (30), der einen nichtflüchtigen Schreib-Lese Speicher umfasst;
wobei das Signalempfangsalement (21) während einer Empfangszeit eine Vielzahl der Paare von physiologischen Signalen empfängt, die durch die mehreren drahtlosen Signalsensoren (10) in einem Einzelfrequenzkanal ausgegeben werden, und die Vielzahl Paare von physiologischen Signalen an das Signalverarbeitungselement (22) überträgt;
wobei das Signalverarbeitungselement (22) die Empfangszeit in dem Signalfrequenzkanal in n gleiche Intervalle teilt und jedes der getrennten Zeitintervalle jedem der drahtlosen Sensoren (10) zuordnet;
wobei das Signalempfangselement (21) eines der Paare von aufgenommenen physiologischen Signalen einem Zeitintervall zuordnet, das dem jeweiligen Sensor (10) entspricht, wenn das Übertragungsmodul (14) des jeweiligen Sensors (10) nicht das aufgenommene Paar von physiologischen Signalen zu dem entsprechenden Zeitintervall sendet;
wobei das Signalempfangselement (21) ferner ein Rückkopplungssignal an das Übertragungsmodul (14) von jedem der mehreren drahtlosen Sensoren (10) sendet, um zu bewirken, dass jedes der Übertragungsmodule (14) das Paar von physiologischen Signalen in einer synchronisierten Weise sendet; und
wobei die verarbeiteten physiologischen Signale in dem Signalaufzeichner (30) gespeichert werden.

12. Verfahren nach Anspruch 11, das ferner einen Signalerkennungsschritt durch jeden der mehreren drahtlosen Signalsensoren (10) umfasst.

13. Verfahren nach Anspruch 11, das bei der Bewertung der Schlafqualität, bei der Diagnose von Schlafstörungen, bei der Einschätzung der Wirkung von Hypnose, bei der Bewertung der Nebenwirkungen auf Schlaf- und autonome Nervenfunktionen, die durch verschiedene Medikamente verursacht werden, bei der Einschätzung des Einflusses auf Schlaf- und autonome Nervenfunktionen durch verschiedene Diät- und Gesundheitsverbesserungsverfahren, bei der Bewertung von Einflüssen auf Schlaf-und autonomen Nervenfunktionen, die durch die Aufnahme von Gesundheitsessen verursacht werden, und bei der Einschätzung der Schlafbedingungen von Älteren und Neugeborenen verwendet werden kann.

## Revendications

1. Appareil pour traiter des signaux physiologiques comprenant :
plusieurs capteurs sans fil (10), chacun comprenant une paire d'électrodes (11), un amplificateur (12), un microcontrôleur (13), un module émetteur (14) et une alimentation de courant (15), la paire d'électrodes (11) étant différentielle et comprenant une électrode positive et une électrode négative, reliées à une personne subissant un test pour recueillir une paire de signaux physiologiques, un dispositif de traitement de signal (20) comprenant :
un élément recevant les signaux (21) et
un élément traitant les signaux (22) et
un enregistreur de signaux (30) comprenant une mémoire vive non volatile, l'élément recevant les signaux (21) étant configuré pour recevoir pendant un temps de réception une pluralité de la paire de signaux physiologiques émis par les plusieurs capteurs sans fil (10) dans un seul canal de fréquences et l'élément recevant les signaux (21) étant configuré pour transmettre la pluralité de la paire de signaux physiologiques à l'unité de traitement de signal (22),
l'élément de traitement de signaux (22) étant configuré pour diviser le temps de réception dans le canal de fréquence de signal en n intervalles égaux et pour faire correspondre chaque intervalle de temps divisé à chacun des plusieurs capteurs (10),
l'élément recevant les signaux (21) étant configuré pour faire correspondre à l'une des paires de signaux physiologiques recueillis à l'intervalle de temps correspondant au capteur respectif (10) si le module émetteur (14) du capteur respectif (10) n'émet pas la paire de signaux physiologiques recueillis à l'intervalle de temps correspondant,
l'élément recevant les signaux (21) étant de plus configuré pour de plus émettre un signal de retour au module émetteur (14) de chacun des plusieurs capteurs sans fil (10) faisant en sorte que chacun des modules émetteurs (14) émette la paire de signaux physiologiques de manière synchronisée et
l'appareil étant configuré pour enregistrer les signaux physiologiques traités dans l'enregistreur de signaux (30).

2. Appareil selon la revendication 1, n étant de l'ordre de 1 à 50.

3. Appareil selon la revendication 1, le capteur de signaux (10) étant sélectionné dans le groupe comportant un capteur d'électrocardiogramme sans fil (104), un capteur d'électro-encéphalogramme sans fil (100), un capteur de température sans fil 103), un capteur d'électromyogramme (102), un capteur de tension miniature (105) et un capteur d'accélération.

4. Appareil selon la revendication 1, le module émetteur (14) étant une interface radio.

5. Appareil selon la revendication 1, ledit enregistreur de signal (30) étant un disque dur, une disquette, un disque dur miniature ou une carte mémoire flash.

6. Appareil selon la revendication 1 qui est exécuté par un système de micro-ordinateur tel qu'un ordinateur personnel (40), un notebook, une station radio ou un assistant numérique personnel (60).

7. Appareil selon la revendication 1 qui peut de plus analyser les données recueillies ou fournir les données recueillies à d'autres éléments récepteurs de signaux.

8. Appareil selon la revendication 7, l'analyse des données étant exécutée par un algorithme d'analyse du sommeil et un algorithme d'analyse du système nerveux autonome.

9. Appareil selon la revendication 1, le dispositif de traitement de signal (20) et les plusieurs capteurs sans fil (10) étant intégrés par un système sur puce

10. Appareil selon la revendication 1, la paire de signaux physiologiques étant un signal physiologique humain.

11. Procédé pour traiter des signaux physiologiques comprenant :
la fourniture de plusieurs capteurs sans fil (10), chacun comprenant une paire d'électrodes (11), un amplificateur (12), un microcontrôleur (13), un module émetteur (14) (14) et une alimentation de courant (15), la paire d'électrodes (11) étant différentielle et comprenant une électrode positive et une électrode négative, reliées à une personne subissant un test pour recueillir une paire de signaux physiologiques,
la fourniture d'un dispositif de traitement de signal (20) comprenant :
un élément recevant les signaux (21) et
un élément traitant les signaux (22) et
la fourniture d'un enregistreur de signaux (30) comprenant une mémoire vive non volatile,
l'élément recevant les signaux (21) recevant pendant un temps de réception une pluralité de la paire de signaux physiologiques émis par les plusieurs capteurs sans fil (10) dans un seul canal de fréquences et transmet la pluralité de la paire des signaux physiologiques à l'élément traitant les signaux (22),
l'élément traitant les signaux (22) divisant le temps de réception dans le seul canal de fréquences en n intervalles égaux et faisant correspondre chaque intervalle de temps divisé à chacun des capteurs sans fil (10) l'élément recevant les signaux (21) faisant correspondre l'une des paires de signaux physiologiques recueillis à l'intervalle de temps correspondant au capteur respectif (10) si le module émetteur (14) du capteur respectif (10) n'émet pas la paire de signaux physiologiques recueillis à l'intervalle de temps correspondant, l'élément recevant les signaux (21) émettant de plus un signal de retour au module émetteur (14) de chacun des plusieurs capteurs sans fil (10) en faisant envoyer par chaque module émetteur (14) la paire de signaux physiologiques de manière synchronisée et les signaux physiologiques traités étant enregistrés dans l'enregistreur de signaux (30).

12. Procédé selon la revendication 11 qui comprend de plus une étape de détection de signal par chacun des plusieurs capteurs sans fil (10).

13. Procédé selon la revendication 11 qui peut être utilisé dans l'évaluation de l'analyse du sommeil, le diagnostic des obstacles au sommeil, l'évaluation de l'effet des hypnotiques, l'évaluation des effets secondaires pour la fonction de sommeil et du système nerveux autonome causés par différents médicaments, l'évaluation des influences sur le sommeil et la fonction du système nerveux autonome dues à différents régimes et procédés d'amélioration de la santé, l'évaluation des influences sur le sommeil et la fonction du système nerveux autonome causées par la prise d'aliments diététiques et l'évaluation de l'état du sommeil des personnes âgées et des nouveaux-nés.
